# EUROPEAN PATENT APPLICATION

(11) **EP 4 678 730 A1**
(43) Date of publication of application: **14.01.2026**
(21) Application number: 24815824.8
(22) Date of filing: 27.05.2024
(51) Int. Cl.: C12M 3/06, C12M 3/00, C12M 1/34

(54) **APPARATUS FOR FORMING MICRO-BIOLOGICAL TISSUE SYSTEM**

(30) Priority: 02.06.2023 KR 20230071908
(71) Applicant: Mepsgen Co., Ltd., Seoul 05836 (KR)
(72) Inventor: RHO, Hoon Suk, Seoul 06344 (KR); KIM, Yong Tae, Seoul 05855 (KR)
(74) Representative: Richly & Ritschel Patentanwälte PartG mbB
(86) International application number: PCT/KR2024/007149
(87) International publication number: WO 2024/248448

(57) **Abstract**

The present invention relates to an apparatus for forming a microphysiological system, including: a cabinet; a culture unit; an injection unit; a perfusion unit; and a resistance measurement unit. Specifically, in the microphysiological system of the present invention, the culture unit has a microfluidic device mounted thereon and inverts the microfluidic device, the injection unit is positioned at an upper end of the culture unit to supply cells and culture medium to the microfluidic device, the perfusion unit is positioned adjacent to the upper end of the culture unit to generate perfusion into an internal channel of the microfluidic device at a constant flow rate, and the resistance measurement unit is positioned at upper and lower ends of the culture unit to measure the resistance between upper and lower channels of the microfluidic device.

## Description

### [Technical Field]

The present invention relates to an apparatus for forming a microphysiological system (MPS). Specifically, the present invention relates to an automated apparatus for forming a microphysiological system, including: a microfluidic device; a culture device for forming a 2D-3D connective tissue or a 3D cell tissue structure within the microfluidic device; an inversion device for changing the position of the microfluidic device between upper and lower orientations; an injection device for injecting cells and culture medium; a perfusion device for activating cell tissues; a gas concentration and temperature control device for creating an environment suitable for cell culture; and a resistance measurement device (transepithelial/transendothelial electrical resistance, TEER) for verifying the formation of the MPS.

### [Background Art]

To address the ethical concerns and cross-species differences associated with using experimental animal models to test drug efficacy and side effects, a microphysiological system (MPS) has been developed, which integrates organ tissues on a chip. The microphysiological system is based on a technology for culturing cells that constitute specific organs on a microfluidic chip, thereby mimicking the morphology and physiological characteristics of the organ and achieving desired functions. This allows for detailed studies of cell behavior and the mechanisms of physicochemical reactions in the microenvironment of specific organs, and may serve as a valuable model for new drug development and toxicity assessment.

In the development of the microphysiological system, three-dimensional (3D) cell culture models implemented on the microfluidic chip closely mimic in vivo conditions, enabling the complexities of directional growth and cell-tocell connections in an in vitro experiment. In addition, these models are useful for studying molecular-based tissue functions, enabling the capture of signaling pathways and drug responsiveness in various disease states, compared to two-dimensional (2D) cell culture models.

However, the process of implementing the microphysiological system using 3D cell culture models requires a high level of technical expertise compared to conventional 2D cell culture models. In particular, the extracellular matrix (ECM) required for 3D cell culture typically generates high shear stress, making it difficult to inject cells into channels within the microfluidic chip. These characteristics not only require a high level of user skill to implement the microphysiological system, but also may lead to variations in implemented systems between users, thereby potentially hindering the statistical significance of test results.

U.S. Patent Publication Nos. 20200332240 and 20210024866, and U.S. Patent Nos. 11597899 and 10519410, all of Emulate, describe a technique for perfusing a culture medium at a constant flow rate through a microfluidic chip using pressure generated by a pump, and a device that provides a cell culture environment for implementing the microphysiological system on the chip. In addition, U.S. Patent Publication No. 20200393397, of Mimetas, describes a device for measuring the resistance (TEER) of a microphysiological system implemented on a microfluidic chip. These technologies may automate some of the processes involved in implementing the microphysiological system, thereby enhancing user convenience and minimizing variation in test results due to differences in technical proficiency between users. However, these technologies only automate some of the processes involved in implementing the microphysiological system and are limited in addressing the above-described variation in test results.

Therefore, the inventors of the present invention have developed equipment that automates all processes required for implementing and verifying the microphysiological system, from the cell injection stage, in order to improve user convenience and minimize deviations between test results in implementing the microphysiological system, thereby completing the present invention.

### (Prior Art Document)

### (Patent Document)

(Patent Document 1) Korean Patent No. 10-2313280
(Patent Document 2) U.S. Patent Publication No. 20200332240
(Patent Document 3) U.S. Patent Publication No. 20210024866
(Patent Document 4) U.S. Patent No. 11597899
(Patent Document 5) U.S. Patent No. 10519410
(Patent Document 6) U.S. Patent Publication No. 20200393397

### (Non-Patent Document)

(Non-Patent Document 1) Cho, H. et al. Three-dimensional blood-brain barrier model for in vitro studies of neurovascular pathology. Sci. Rep. 5, 15222 (2015).
(Non-Patent Document 2) Booth, R. & Kim, H. Characterization of a microfluidic in vitro model of the blood-brain barrier (µBBB). Lab Chip 12, 1784-1792 (2012).

### [Summary of Invention]

### [Problems to be Solved by Invention]

An object of the present invention is to provide an apparatus for forming a microphysiological system including: a microfluidic device; a culture device for forming a cell tissue structure within the microfluidic device; an inversion device for flipping the position of the microfluidic device between upper and lower orientations; an injection device for injecting cells and culture medium; a perfusion device for activating cell tissues; a gas concentration and temperature control device for creating an environment suitable for cell culture; and a resistance measurement device for verifying the formation of the microphysiological system.

In addition, another object of the present invention is to provide an apparatus for forming a microphysiological system that automates the entire process required for implementing and verifying the microphysiological system.

### [Means for Solving Problems]

The present invention provides an apparatus for forming a microphysiological system including: a microfluidic device; a culture unit; an injection unit; a perfusion unit; and a resistance measurement unit.

The culture unit may include at least one of a microfluidic device mounting unit, a microfluidic device inversion driving unit, and an introduction unit.

The injection unit may include at least one of an injection unit horizontal movement device, an injection unit vertical movement device, a syringe vertical movement device, and a syringe.

The perfusion unit may include at least one of a perfusion unit vertical movement device and a perfusion unit injection connection part.

The resistance measurement unit may include a resistance measurement unit upper portion and a resistance measurement unit lower portion. The resistance measurement unit upper portion may include a vertical movement device and a resistance measurement unit upper pin connection part, and the resistance measurement unit lower portion may include a vertical movement device and a resistance measurement unit lower pin connection part.

The apparatus for forming a microphysiological system may be installed inside a cabinet, and the inside of the cabinet may be maintained at a constant gas concentration or temperature.

The apparatus for forming a microphysiological system may measure the formation and activation of a microphysiological structure inside the microfluidic device.

### [Advantageous Effects]

The apparatus for forming a microphysiological system of the present invention may sequentially and consistently supply cells, hydrogel, and culture medium into a microfluidic device to form perfusion within the channel of the microfluidic device, thereby activating cell tissues. In addition, the inside of the apparatus may be maintained at a constant gas concentration and temperature, thereby creating an environment suitable for cell culture. Further, by connecting electrodes to the microfluidic device, the electrical resistance passing through the tissue barrier formed within the microfluidic device may be measured to verify the tissue barrier.

Therefore, the apparatus of the present invention automates all processes necessary for the implementation and verification of the microphysiological system, thereby enhancing user convenience and minimizing variations in test results due to differences in technical proficiency between users.

### [Brief Description of Drawings]

FIG. 1 is a perspective view of an apparatus for forming a microphysiological system.
FIG. 2 is a perspective view of the apparatus for forming a microphysiological system with a door open.
FIG. 3 is a perspective view illustrating a state in which a culture unit, an injection unit, a perfusion unit, and a resistance measurement unit are installed in the apparatus for forming a microphysiological system.
FIG. 4 is a perspective view of the culture unit.
FIG. 5 is a plan view of the culture unit.
FIG. 6 is a bottom view of the culture unit.
FIG. 7 is a perspective view of a microfluidic device mounting unit.
FIG. 8 is a perspective view illustrating a state in which a microfluidic device mounted on the culture unit is inverted between upper and lower orientations.
FIG. 9 is a perspective view of the culture unit and the injection unit.
FIG. 10 is a perspective view of the culture unit and the perfusion unit.
FIG. 11 is a side view of the culture unit and the perfusion unit.
FIG. 12 is a perspective view of the culture unit and the resistance measurement unit.
FIG. 13 is a side view of the culture unit and the resistance measurement unit.

### [Mode for Carrying out Invention]

Hereinafter, embodiments and examples of the present invention will be described in detail with reference to the accompanying drawings, so that those skilled in the art can readily practice the present invention. However, it should be understood that the present invention may be embodied in various forms and is not limited to the specific embodiments and examples described herein.

Throughout this specification, when a part is referred to as "including" a component, it should be understood that, unless explicitly stated otherwise, the term does not exclude the presence of other components but may further include additional components.

The present invention relates to an apparatus for forming a microphysiological system, including a microfluidic device, a culture unit, an injection unit, a perfusion unit, and a resistance measurement unit.

The apparatus for forming a microphysiological system may measure the formation and activation of a microphysiological structure within the microfluidic device.

In one embodiment, the culture unit, the injection unit, the perfusion unit, and the resistance measurement unit may be installed inside a cabinet, the microfluidic device may be mounted on the culture unit, cells and hydrogel may be introduced into the culture unit, the cells and hydrogel may be injected into the microfluidic device through the injection unit, the perfusion unit may be connected to the microfluidic device and may inject the culture medium at a constant rate, and the resistance measurement unit may be connected to the upper and lower portions of the microfluidic device to measure an electrical resistance value.

As used herein, the term "microfluidic device" refers to a device including a microchannel configured to allow a fluid to flow on a substrate made of various materials, including plastic, glass, metal, or silicon, and which may include an organic polymer.

As used herein, the term "microchannel" refers to a channel having dimensions on the order of millimeters, micrometers, or nanometers, through which a fluid can flow, and is also referred to as a "mixing channel" in the present invention.

The term "cell" as used herein refers to biological cells, including plant cells, animal cells (e.g., mammalian cells), bacterial cells, and fungal cells.

The term "tissue barrier cell" as used herein refers to a cell that maintains the specific structure of a tissue and protects the tissue from external stimuli. In addition, it refers to a cell that utilizes strong binding between tissue barrier cells or with the extracellular matrix to selectively allow substances to pass through and maintain homeostasis in the concentration of substances within the tissue.

The term "hydrogel" as used herein refers to a material having a 3D polymer network structure that is cross-linked by cohesive forces such as covalent bonds, hydrogen bonds, van der Waals bonds, or physical bonds and may swell by containing a large amount of water inside an aqueous solution.

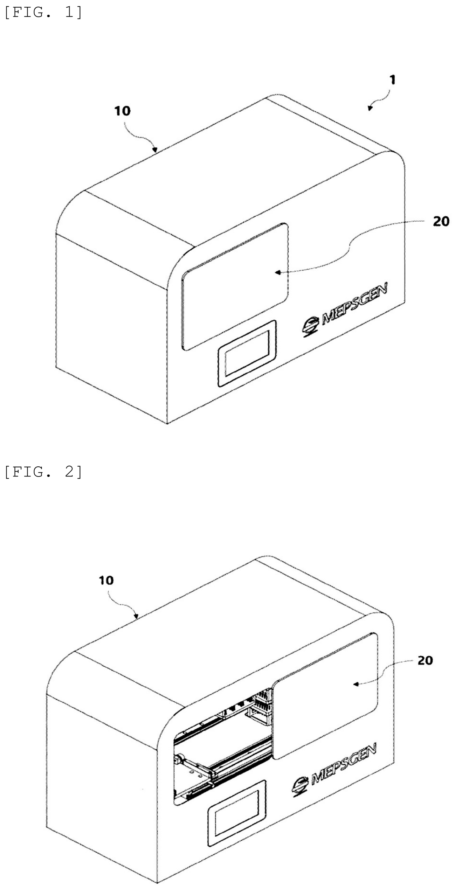

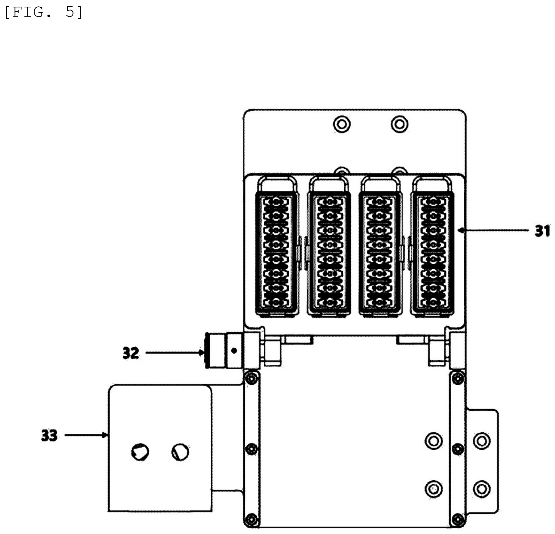

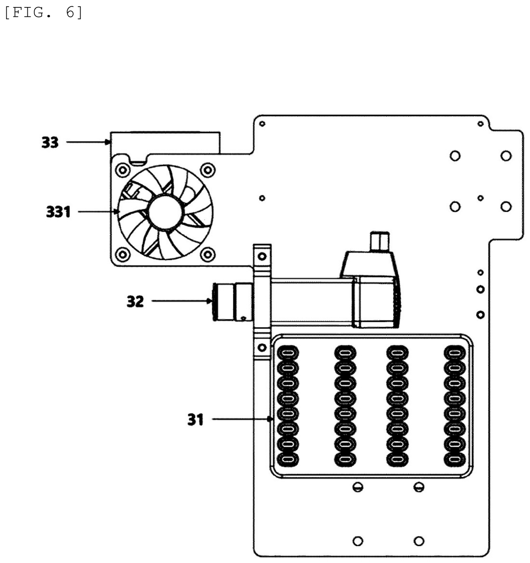

Hereinafter, an apparatus for forming a microphysiological system of the present invention will be described in detail with reference to the drawings. The present invention may include an apparatus for forming a microphysiological system embodied in the form of FIGS. 1 to 13, but is not limited thereto, and may encompass various modifications implemented by those skilled in the art to which the present invention pertains.

An apparatus 1 for forming a microphysiological system of the present invention includes a culture unit 30, an injection unit 40, a perfusion unit 50, and a resistance measurement unit 60.

Referring to FIGS. 1 and 2, the exterior of the apparatus 1 for forming a microphysiological system of the present invention may be formed by a cabinet 10 in which a microphysiological system is formed, and an openable and closable door 20.

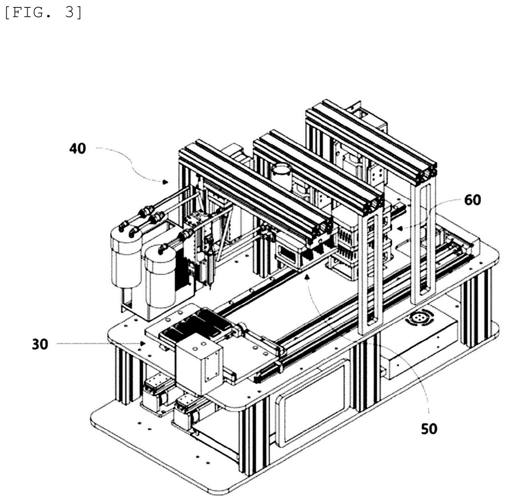

Referring to FIG. 3, the culture unit 30, the injection unit 40, the perfusion unit 50, and the resistance measurement unit 60 may be installed inside the cabinet 10. In addition, the inside of the cabinet may be maintained at a constant gas concentration or temperature.

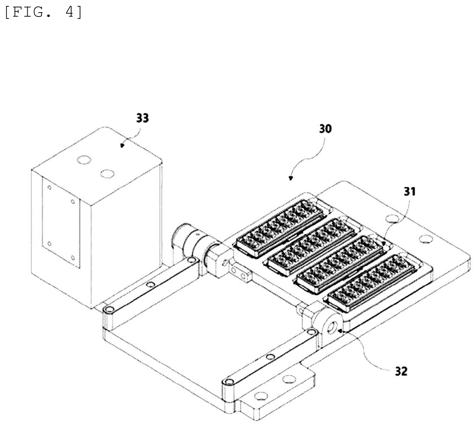

Referring to FIG. 4 to 6, the culture unit 30 may include a microfluidic device mounting unit 31, a microfluidic device inversion driving unit 32, and an introduction unit 33. In addition, the microfluidic device mounting unit 31 may be connected to the microfluidic device inversion driving unit 32, the introduction unit 33 may be provided on a side of the microfluidic device mounting unit 31, and the introduction unit 33 may include a cooling device 331 for temperature control.

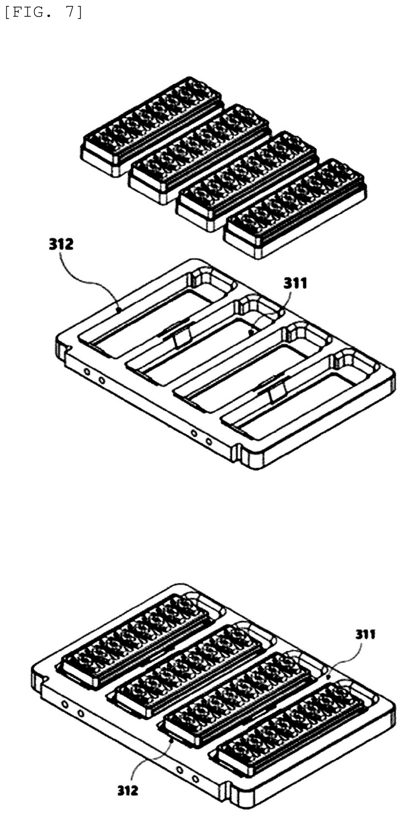

Referring to FIG. 7, the microfluidic device mounting unit 31 may include a bed 311 on which one or two or more microfluidic devices may be mounted, and a fixing clip 312 for fixing the microfluidic devices.

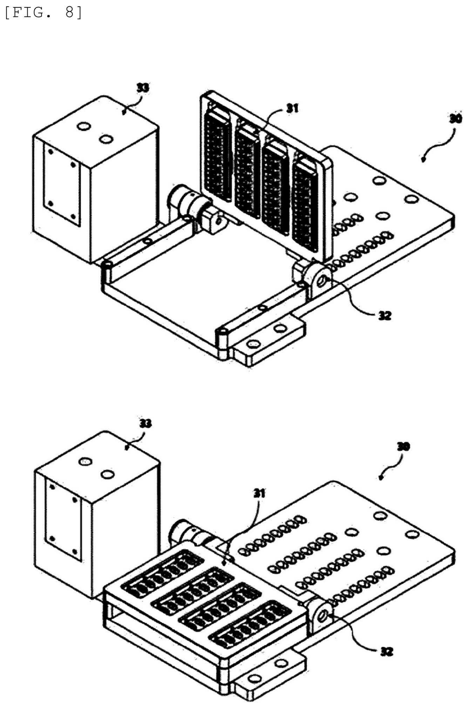

Referring to FIG. 8, the microfluidic device mounting unit 31 may be rotated by the microfluidic device inversion driving unit 32, the microfluidic device mounting unit 31 may be inverted between upper and lower orientations by the microfluidic device inversion driving unit 32, and the inversion may be repeated one or more times.

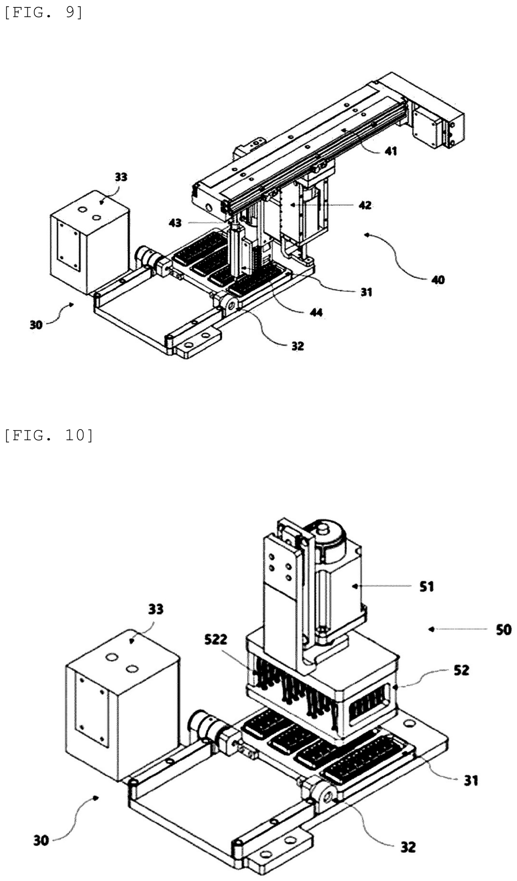

Referring to FIG. 9, the injection unit 40 may include an injection unit horizontal movement device 41, an injection unit vertical movement device 42, a syringe vertical movement device 43, and a syringe 44. In addition, the injection unit 40 may inject a substance introduced into the introduction unit 33 through the injection unit horizontal movement device 41 and the injection unit vertical movement device 42 into the syringe 44, and the injection unit 40 may inject a substance contained in the syringe 44 through the injection unit horizontal movement device 41 and the injection unit vertical movement device 42 into the microfluidic device. The substance may be at least one selected from the group consisting of cells, hydrogels, cell culture medium, and organic solvents.

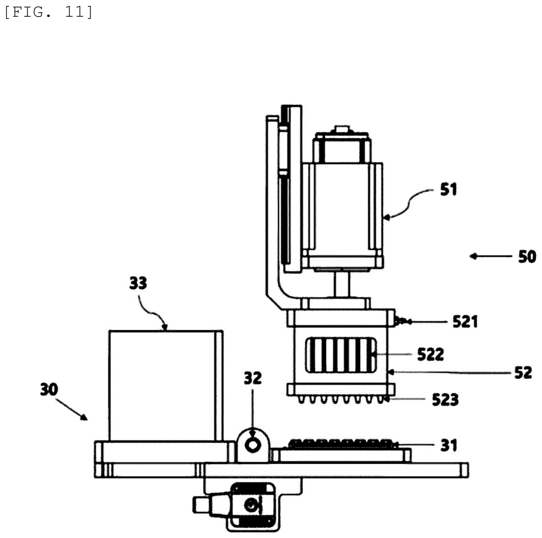

Referring to FIGS. 10 and 11, the perfusion unit 50 may include a perfusion unit vertical movement device 51 and a perfusion unit injection connection part 52. The perfusion unit 50 may inject a culture medium into the microfluidic device by connecting the perfusion unit injection connection part 52 to the microfluidic device mounted on the culture unit 30 through the perfusion unit vertical movement device 51, and may generate perfusion at a constant flow rate in the internal channel of the microfluidic device. In addition, the perfusion unit injection connection part 52 may include: one or two or more injection connection nozzles 521; a connection tube 522; and a microfluidic device connection nozzle 523, and may inject the culture medium stored in the culture medium reservoir into the microfluidic device through the injection connection nozzle 521, the connection tube 522, and the microfluidic device connection nozzle 523.

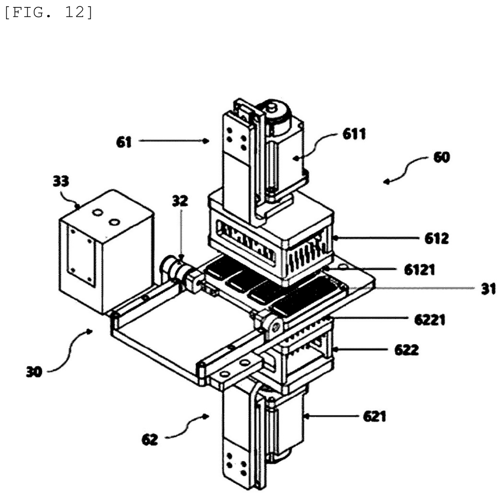

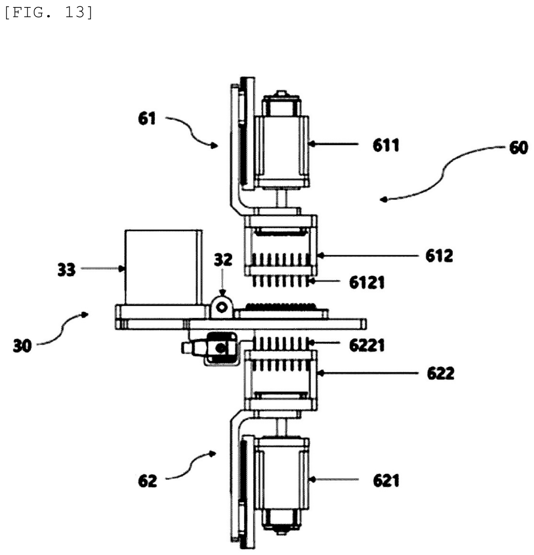

Referring to FIGS. 12 and 13, the resistance measurement unit 60 may include a resistance measurement unit upper portion 61 and a resistance measurement unit lower portion 62. The resistance measurement unit upper portion 61 may include a vertical movement device 611 and a resistance measurement unit upper pin connection part 612, and the resistance measurement unit lower portion 62 may include a vertical movement device 621 and a resistance measurement unit lower pin connection part 622. In addition, the resistance measurement unit upper pin connection part 612 may include one or two or more resistance measurement unit upper pins 6121, and the resistance measurement unit lower pin connection part 622 may include one or two or more resistance measurement unit lower pins 6221. The resistance measurement unit upper pin connection part 612 may move to an upper portion of a microfluidic device upper inlet through the vertical movement device 611 to connect the resistance measurement unit upper pin 6121 to the microfluidic device inlet, and the resistance measurement unit lower pin connection part 622 may move to a lower portion of the microfluidic device lower inlet through the vertical movement device 621 to connect the resistance measurement unit lower pin 6221 to the microfluidic device inlet, and may measure an electrical resistance value through a tissue barrier formed in the microfluidic device.

### [Description of Reference Numerals]

1: Apparatus for forming a microphysiological system
10: Cabinet
20: Door
30: Culture unit
31: Microfluidic device mounting unit
311: Bed
312: Fixing clip
32: Microfluidic device inversion driving unit
33: Introduction unit
331: Cooling device
40: Injection unit
41: Injection unit horizontal movement device
42: Injection unit vertical movement device
43: Syringe vertical movement device
44: Syringe
50: Perfusion unit
51: Perfusion unit vertical movement device
52: Perfusion unit injection connection part
60: Measurement part
61: Resistance measurement unit upper portion
611: Vertical movement device
612: Resistance measurement unit upper pin connection part
6121: Resistance measurement unit upper pin
62: Resistance measurement unit lower portion
621: Vertical movement device
622: Resistance measurement unit lower pin connection part
6221: Resistance measurement unit lower pin

## Claims

1. An apparatus for forming a microphysiological system comprising: a microfluidic device; a culture unit; an injection unit; a perfusion unit; and a resistance measurement unit.

2. The apparatus for forming a microphysiological system according to claim 1, wherein the culture unit comprises at least one of a microfluidic device mounting unit, a microfluidic device inversion driving unit, and an introduction unit.

3. The apparatus for forming a microphysiological system according to claim 2, wherein the microfluidic device mounting unit is connected to the microfluidic device inversion driving unit, and the introduction unit is provided on a side of the microfluidic device mounting unit.

4. The apparatus for forming a microphysiological system according to claim 2, wherein the microfluidic device mounting unit comprises: a bed on which one or two or more microfluidic devices are mounted; and a fixing clip configured to fix the microfluidic devices.

5. The apparatus for forming a microphysiological system according to claim 2, wherein the microfluidic device mounting unit is rotated by the microfluidic device inversion driving unit.

6. The apparatus for forming a microphysiological system according to claim 5, wherein the microfluidic device mounting unit is inverted between its upper and lower orientations by the microfluidic device inversion driving unit, and the inversion can be repeated one or more times.

7. The apparatus for forming a microphysiological system according to claim 2, wherein the introduction unit comprises a cooling device for temperature control.

8. The apparatus for forming a microphysiological system according to claim 1, wherein the injection unit comprises at least one of an injection unit horizontal movement device, an injection unit vertical movement device, a syringe vertical movement device, and a syringe.

9. The apparatus for forming a microphysiological system according to claim 8, wherein the injection unit injects a substance, which has been introduced into introduction unit, into the syringe via the injection unit horizontal movement device and the injection unit vertical movement device.

10. The apparatus for forming a microphysiological system according to claim 9, wherein the injection unit injects a substance contained in the syringe into the microfluidic device through the injection unit horizontal movement device and the injection unit vertical movement device.

11. The apparatus for forming a microphysiological system according to claim 9, wherein the substance is at least one selected from the group consisting of cells, hydrogels, cell culture medium, and organic solvents.

12. The apparatus for forming a microphysiological system according to claim 1, wherein the perfusion unit comprises at least one of a perfusion unit vertical movement device and a perfusion unit injection connection part.

13. The apparatus for forming a microphysiological system according to claim 12, wherein the perfusion unit injection connection part comprises at least one of one or two or more injection connection nozzles, a connection tube, and a microfluidic device connection nozzle.

14. The apparatus for forming a microphysiological system according to claim 12, wherein the perfusion unit connects the perfusion unit injection connection part to the microfluidic device mounted on the culture unit through the perfusion unit vertical movement device, thereby injecting culture medium into the microfluidic device.

15. The apparatus for forming a microphysiological system according to claim 12, wherein the perfusion unit generates perfusion at a constant flow rate in the internal channel of the microfluidic device mounted on the culture unit.

16. The apparatus for forming a microphysiological system according to claim 1, wherein the resistance measurement unit comprises a resistance measurement unit upper portion and a resistance measurement unit lower portion, the resistance measurement unit upper portion comprising a vertical movement device and a resistance measurement unit upper pin connection part, and the resistance measurement unit lower portion comprising a vertical movement device and a resistance measurement unit lower pin connection part.

17. The apparatus for forming a microphysiological system according to claim 16, wherein the resistance measurement unit upper pin connection part comprises one or two or more resistance measurement unit upper pins, and the resistance measurement unit lower pin connection part comprises one or two or more resistance measurement unit lower pins.

18. The apparatus for forming a microphysiological system according to claim 16, wherein the resistance measurement unit upper pin connection part moves to an upper portion of a microfluidic device upper inlet through the vertical movement device to connect the resistance measurement unit upper pin to the microfluidic device inlet, and the resistance measurement unit lower pin connection part moves to a lower portion of the microfluidic device lower inlet through the vertical movement device to connect the resistance measurement unit lower pin to the microfluidic device inlet, thereby measuring an electrical resistance through a tissue barrier formed in the microfluidic device.

19. The apparatus for forming a microphysiological system according to claim 1, wherein the apparatus for forming a microphysiological system is installed inside a cabinet.

20. The apparatus for forming a microphysiological system according to claim 19, wherein the inside of the cabinet is maintained at a constant gas concentration or temperature.

21. The apparatus for forming a microphysiological system according to claim 1, wherein the apparatus for forming a microphysiological system measures the formation and activation of a microphysiological structure within the microfluidic device.
